# EUROPÄISCHE PATENTSCHRIFT

(11) **EP 1 866 423 B1**
(45) Veröffentlichungstag und Bekanntmachung des Hinweises auf die Patenterteilung: **25.01.2012**
(21) Anmeldenummer: 06704767.0
(22) Anmeldetag: 31.03.2006
(51) Int. Cl.: C12P 1/02, C12P 1/04

(54) **Herstellung von hochgradig isotopenmarkierten, sekundären, mikrobiellen Stoffwechselprodukten sowie entsprechende Stoffwechselprodukte**
Production of highly isotopically labelled, secondary, microbial metabolic products, and corresponding metabolic products
Production de métabolites microbiens secondaires, hautement marquées d'un isotope et metabolites correspondants

(30) Priorität: 05.04.2005 AT 5742005
(43) Veröffentlichungstag der Anmeldung: 19.12.2007
(73) Patentinhaber: Erber Aktiengesellschaft, 3130 Herzogenburg (AT)
(72) Erfinder: FREUDENSCHUSS, Martin, A-3370 Ybbs (AT); HÄUBL, Georg, A-1050 Wien (AT); KRSKA, Rudolf, A-3430 Tulln (AT); JAUNECKER, Günther, A-3430 Tulln (AT); BINDER, Eva, A-1190 Wien (AT)
(74) Vertreter: Cunow, Gerda
(86) Internationale Anmeldenummer: PCT/AT2006/000129
(87) Internationale Veröffentlichungsnummer: WO 2006/105563

(56) Entgegenhaltungen:
- WO-A1-00/37665
- WO-A1-00/37665
- KURZATKOWSKI W ET AL: "RADIOACTIVE PENICILLIN G PRODUCTION BY IMMOBILIZED FUNGAL VESICLES" APPLIED MICROBIOLOGY AND BIOTECHNOLOGY, Bd. 19, Nr. 5, 1984, Seiten 312-315, XP008067245 ISSN: 0175-7598
- LINDENMEIER M ET AL: "Quantification of ochratoxin A in foods by a stable isotope dilution assay using high-performance liquid chromatography-tandem mass spectrometry" JOURNAL OF CHROMATOGRAPHY, ELSEVIER SCIENCE PUBLISHERS B.V. AMSTERDAM, NL, Bd. 1023, Nr. 1, 9. Januar 2004 (2004-01-09), Seiten 57-66, XP004476678 ISSN: 0021-9673
- CARSON D B ET AL: "BIODEGRADATION OF N-PHOSPHONOMETHYLIMINODIACETIC ACID BY MICROORGANISMS FROM INDUSTRIAL ACTIVATED SLUDGE" CANADIAN JOURNAL OF MICROBIOLOGY, OTTAWA, CA, Bd. 43, Nr. 1, 1997, Seiten 97-101, XP008038399 ISSN: 0008-4166
- LADD J N ET AL: "Distribution of organic 14C and 15N in particle-size fractions of soils incubated with 14C, 15-labelled glucose/NH-4, and legume and sheat straw residues" SOIL BIOLOGY AND BIOCHEMISTRY, Bd. 28, Nr. 7, 1996, Seiten 893-905, XP002393191 ISSN: 0038-0717
- HAEUBL G ET AL: "Suitability of a fully C-13 isotope labeled internal standard for the determination of the mycotoxin deoxynivalenol by LC-MS/MS without clean up" ANALYTICAL AND BIOANALYTICAL CHEMISTRY, Bd. 384, Nr. 3, Februar 2006 (2006-02), Seiten 692-696, XP002393192 ISSN: 1618-2642
- 'Stable isotope labeled mycotoxin calibrants' BIOPURE REFERENZSUBSTANZEN GMBH, [Online] Gefunden im Internet: <URL:www.biopure.at/biopure-index/products/ is.htm>
- BLACKWELL B A ET AL: 'CARBON-13 NMR STUDY OF THE BIOSYNTHESIS OF TOXINS BY FUSARIUM-GRAMINEARUM', 1985, JOURNAL OF BIOLOGICAL CHEMISTRY, VOL. 260, NR. 7, PAGE(S) 4243-4247, ISSN 0021-9258 Seiten 4243 - 4247
- 'Stable isotope labeled mycotoxin calibrants' BIOPURE REFERENZSUBSTANZEN GMBH, [Online] Gefunden im Internet: <URL:www.biopure.at/biopure-index/products/ is.htm>
- BLACKWELL B A ET AL: "CARBON-13 NMR STUDY OF THE BIOSYNTHESIS OF TOXINS BY FUSARIUM-GRAMINEARUM", 1985, JOURNAL OF BIOLOGICAL CHEMISTRY, VOL. 260, NR. 7, PAGE(S) 4243-4247 ISSN: 0021-9258

## Beschreibung

Die vorliegende Erfindung bezieht sich auf ein Verfahren zur Herstellung von isotopenmarkierten, sekundären Stoff-wechselprodukten von Pilzen oder Bakterien in einem flüssigen, synthetischen Kulturmedium sowie auf isotopenmarkierte, sekundäre Stoffwechselprodukte gewählt aus der Gruppe Mycotoxine, Toxine oder Antibiotika.

Isotopenmarkierte Substanzen gewinnen in der heutigen Zeit immer größere Bedeutung, insbesondere in der Technologie der Flüssigkeitschromatografie mit massenspektrometrischer Detektion (LCMS), in welcher eine effiziente, spektrometrische Analyse mit hohem Produktdurchsatz ermöglicht wird. Die Technologie kann für eine große vielfalt von potentiellen Analyten eingesetzt werden, wobei es keine Begrenzung der Molekülmasse gibt, jedoch Probleme bei dem Nachweis der einzelnen Substanzen dahingehend auftreten können, daß sowohl die Zerfallsspektren als auch die einzelnen Molekülpeaks entsprechend zugeordnet werden müssen. Um hiebei eine verläßliche LCMS-Applikation und -Methoden zu erzielen, ist die Verwendung von sogenannten internen Standardsubstanzen von steigender Bedeutung. Interne Standards sind Substanzen, die eine sehr hohe Ähnlichkeit mit dem eigentlichen Zielanalyten aufweisen, d.h. insbesondere wenn möglich eine idente molekulare Struktur aufweisen, jedoch mit unterschiedlichem Molekulargewicht. Als ideale interne Standards erweisen sich daher isotopenmarkierte Moleküle des Zielanalyten, d.h. Moleküle, in welchen ein oder mehrere Atome im Molekül durch ihre Isotopen ersetzt werden. Gegenwärtig werden derartige Substanzen durch organische Synthese hergestellt, indem beispielsweise Wasserstoffe oder Kohlenstoffe durch die entsprechenden schwereren Isotopen ersetzt werden.

In diesem Zusammenhang hat es sich jedoch bei LCMS-Analysen gezeigt, daß es wünschenswert ist, daß die isotopenmarkierten Substanzen, die als interne Standards verwendet werden, einen Molekülmassenunterschied von wenigstens 3 besitzen, um eine deutliche Trennung von dem Zielanalyten erzielen zu können und daß, wenn möglich Substanzen zum Einsatz gelangen sollen, die aus so wenigen Isotopomeren wie möglich bestehen.

Ein Weg, isotopenmarkierte, pflanzliche oder mikrobielle Metaboliten herzustellen, ist über den Biosyntheseweg der entsprechenden Pflanzen und/oder Mikroben. Hiebei werden die Kulturmedien mit radioaktiv markierten Nährstoffen versetzt und die Nährmediumsbestandteile werden zu einem gewissen Prozentsatz in die anabolischen und metabolischen Kreisläufe der Mikroben- oder Pflanzenkulturen eingebaut, so daß Isotope in die Stoffwechselprodukte inkorporiert werden. Ein Nachteil dieses Verfahrens ist es, daß mit diesem Verfahren lediglich eine unvollständige Markierung erzielbar ist und normalerweise ein Gemisch von verschiedensten Isotopomeren gebildet wird, die Verwendung von derartigen isotopenmarkierten Substanzen bzw. isotopenmarkierten, pflanzlichen oder mikrobiellen Metaboliten zur Verwendung als interne Standards nur schlecht geeignet scheint, da bei Einsatz derartiger substanzen nicht ein Standard zur Anwendung käme, sondern eine breite Palette von Isotopomeren, wodurch wiederum ein gezielter Nachweis von Zielsubstanzen in LCMS-Spektrometrien nicht bzw. nur äußerst schwer möglich erscheint.

Der Literaturstelle Wu et al., Analytical Biochemistry 336 (2005) 164-171 ist bereits ein Verfahren zur quantitativen Analyse, der mikrobiellen Metabolome unter Verwendung einer Mischung von vollständig gleichmäßig ¹³C markierten Metaboliten als interne Standards in dem Metabolitenextraktions-verfahren und nachfolgender LC-ESI-MS/MS-Analyse entnehmbar, wobei die vollständigen ¹³C metaboliten aus Saccharomyces cerevisiae kultiviert wurden.

Der Literaturstelle Evans et al., Analytical Biochemistry 306, 197-203 (2002), ist bereits ein LC/MS-Analyseverfahren der NAD Biosynthese unter Verwendung von stabilen, isotopenmarkierten Pyridinvorläufern entnehmbar. In diesem Verfahren wird ein von Hefe abgeleiteter, interner Standard, welcher vollständig in bezug auf die ¹³C und ¹⁵N Atome markiert ist, eingesetzt.

Aus Kurzatkowski et al., Appl Microbiol Biotechnol (1984) 19:312-315, ist die Herstellung von radioaktiv markiertem Penizillin G durch Immobilisierte Pilzveszikel bekannt geworden, mit dem Ziel, die Wirkaktivität bei der Herstellung von radioaktivem Penizillin G aus ¹⁴C-Valin zu messen.

Weiters ist Carson et al., Can. J. Microbiol. 43:97-101 (1997), die Biodegradation von N-Phosphonomethyliminodiessigsäure mittels Mikroorganismen aus industriell aktiviertem Schlamm entnehmbar, wobei die Schlüsselkomponente in diesem Abbauverfahren Glyphosat darstellt, welches ¹⁴C-markiert wurde, um den Abbauprozeß verfolgen zu können.

Dem Dokument Blackwell B A et al: "Carbon-13 NMR Study of the biosynthesis of Toxins by fusarium-graminearum", 1985, Journal of biological chemistry, Band 260, Nr. 7, Seite(n) 4243-4247 ist eine ¹³C-NMR-Studie der Biosynthese von Toxinen durch Fusarium Graminearum entnehmbar. Hiebei werden ¹³C-NMR-Studien betreffend die Biosynthese von Mykotoxinen, die durch Fusarium Graminearum gebildet werden, durch das Inkorporieren von [1-¹³C]- und [2-¹³C]-Acetatvorläufern untersucht.

Dem Dokument Lindenmeier M et al., Journal of Chromatography, Elsevier Science Publiciates B.V., Amsterdam NL, Band 1023, Nr. 1, 9. Jänner 2004, Seiten 47-56 ist eine Quantifizierung von Ochratoxin A in Nahrungsmitteln durch einen stabilen Isotopenverdünnungaversuch unter Verwendung von HPLC, Chromatographie-Tandem-Massenspektrometrie entnehmbar. In dieser Studie wurde ein Istopenverdünnungsversuch zur Quantifizierung des Mykrotoxin Ochratoxin A unter Verwendung von [²H5]-OTA als interner Standard entwickelt.

Die vorliegende Erfindung zielt nun darauf ab, ein Verfahren zur Herstellung von isotopenmapkierten, sekundären Stoffwechselprodukten von Pilzen oder Bakterien zur Verfügung zu stellen, in welchen sämtliche bzw. nahezu sämtliche Kohlenstoffatome, Stickstoffatome oder Schwefelatome in dem Ausgangsprodukt durch stabile Isotopen ersetzt werden und dadurch ein einziges, isotopenrnarkiertes Endprodukt erzielt wird, welches in spektrometrischen Verfahren, insbesondere LCMS, leicht und sicher nachweisbar ist. Die Erfindung zielt weiters auf die Herstellung eines Stoffwechselprodukts ab, welches als interner Standard bzw. in spektrometrischen Analyseverfahren, insbesondere LCMS, sicher und zuverlässig eingesetzt bzw. verwendet werden kann.

Zur Lösung dieser Aufgaben wird das Verfahren gemäß der vorliegenden Erfindung so geführt, daß die Synthese durch Immobilisieren der Pilze oder Bakterien auf einem inerten Träger unter Zugabe von flüssigem, synthetischem Kulturmedium, in welchem wenigstens 95 % der Kohlenstoffatome, Stickstoffatome und/oder Schwefelatome durch stabile Isotope ersetzt wurden, durchgeführt wird. Dadurch, daß die Synthese durch Immobilisieren der Pilze oder Bakterien auf einem inerten Träger unter Zugabe von flüssigem, synthetischem Kulturmedium, in welchem im wesentlichen sämtliche bzw. wenigstens 95 % der Kohlenatoffatome, Stickstoffatome und/oder Schwefelatome durch stabile Isotope ersetzt wurden, durchgeführt wird, gelingt es, ein isotopenmarkiertes Stoffwechselprodukt der Pilze und Bakterien herzustellen, in welchem sämtliche Atome bzw. wenigstens 95 % der Atome, die durch Anzucht aus dem Kulturmedium entnommen werden können, nämlich Kohlenstoff-, Stickstoff- oder Schwefelatome, durch die stabilen Isotopen der im Kulturmedium enthaltenen, isotopenmarkierten Nährsubstanzen ersetzt sind und somit ein gezieltes, isotopenmarkiertes Produkt erhalten werden kann und nicht, wie dies im Stand der Technik vielfach beschrieben wurde, eine Mischung von verschiedenen Homologen mit variierender Anzahl von Isotopenatomen erzielbar ist. Durch dieses Herstellungsverfahren kann somit ein isotopenmarkiertes, sekundäre Stoffwechselprodukt erzielt werden, welches gezielt einsetzbar ist und welches in sämtlichen Analysen bzw. auch in metabolischen Studien klar und eindeutig nachgewiesen werden kann.

Gemäß einer Weiterbildung ist das Verfahren so geführt, daß als Kohlenstoffquelle im flüssigen, synthetischen Kulturmedium Zucker oder Zuckeralkohole, insbesondere D- [U-¹³C₆]-Glucose, ¹³C-Saccharose, ¹³C-Glycerin und/oder ¹³C-Acetat, als Stickstoffquellen ¹⁵N-Aminosläuren, -Nitrate, -Ammoniumverbindungen oder -Harnstoff, als Schwefelquellen ³³S- oder ³⁴S-Sulfate, -Sulfide oder -Aminosäuren eingesetzt sind. Indem vollständig durchmarkierte kohlenstoff-, Stick-stoff- und/oder Schwefelquellen in de flüssigen, synthetischen Kulturmedium enthalten sind, ist bei der Anzucht von Stoffwechselprodukten von Pilzen oder Bakterien der Pilz bzw. das Bakterium gezwungen, in das Stoffwechselprodukt das jeweilige markierte Isotop einzubauen, so daß sichergestellt werden kann, daß die sekundären Stoffwechselprodukte der Pilze oder Bakterien zu einem hohen Grad, wenn nicht vollständig mit den entsprechenden Isotopen markiert bzw. durch die entsprechenden Isotopen ersetzt sind.

Zur Verbesserung der Ausbeute an isotopenmarkierten, sekundären Stoffwechselprodukten von Pilzen oder Bakterien wird das Verfahren gemäß einer Weiterbildung so geführt, daß das flüssige, synthetische Kulturmedium zusätzlich eine Mischung, gewählt aus anorganischen Salzen oder Säuren und Basen mit den Ionen Na⁺, K⁺, Ca⁺⁺, Mg⁺⁺, Fe⁺⁺⁺, Zn⁺⁺, Cu⁺⁺, B⁺⁺⁺ sowie CO₃⁻⁻, SO₄⁻⁻, PO₄⁻⁻⁻, NO₃⁻, enthält Indem in dem flüssigen, synthetischen Kulturmedium Salze oder Säuren und Basen mit den Ionen Na⁺, K⁺, Ca⁺⁺, Mg⁺⁺, Fe⁺⁺⁺, Zn⁺⁺, Cu⁺⁺, B⁺⁺⁺ sowie CO₃⁻⁻, SO₄⁻⁻, PO₄⁻⁻⁻, NO₃⁻ enthalten sind, wird sichergestellt, daß sämtliche Fremdionen, welche möglicherweise in den Pilzen oder Bakterien neben Kohlenstoff, Wasserstoff, Stickstoff und Schwefel enthalten sind, in ausreichender Menge und sicher zur Verfügung gestellt werden, wodurch neben rascher Anzucht auch eine hohe Ausbeute erzielbar ist.

Zur weiteren Verbesserung der Ausbeute wird als inerter. Träger ein natürlicher oder synthetischer Träger mit großer innerer Oberfläche, insbesondere Silikat, Schichtsilikat, Zeolith, Bentonit, gebrannter Ton, Diatomeenerde, Kunststoffe oder dgl., eingesetzt. Durch Einsatz eines inerten Trägers mit großer, innerer Oberfläche gelingt eine Ausbauteverbesserung bei dem Verfahren gemäß der vorliegenden Erfindung von wenigstens 50 % gegenüber herkömmlichen Verfahren, welche ohne inertem Träger mit großer, innerer Oberfläche durchgeführt werden, zu erzielen. Derartige Ausbeutesteigerungen machen das Verfahren nicht nur wirtschaftlicher, sondern stellen auch sicher, daß ausreichende Mengen der gewünschten Endprodukte der isotopenmarkierten, sekundären Stoffwechselprodukte hergestellt werden können, um diese sinnvoll in Analysen als interner Standard bzw. auch in metabolischen Studien einsetzen zu können.

Die größten Verbesserungen der Ausbeute können gemäß der Erfindung insbesondere dadurch erzielt werden, daß als inerter Träger mit großer, innerer Oberfläche ein Aluminiumsilikat, beispielsweise Diatomeenerde, insbesondere Kieselgur, Isolute HM-N oder ein Zeolith, oder ein Schichtsilikat, insbesondere ein Vermiculit, aus der Gruppe der Glimmerminerale, in natürlicher oder behandelter Form eingesetzt wird. Bei diesen Substanzen ist insbesondere die Oberflächenbeschaffenheit, wie Oberflächenspannung, Porosität und dgl., für einen besonders guten Umsatz an der Trägeroberfläche geeignet. In analoger Weise können durch Verwendung inerter Kunststoffträger, die aus Schaumstoff, Polyamid, Silikon, Polyethylen, Polypropylen, Polytetrafluorethylen, Polyester oder dgl., gewählt sein können, entsprechende Ausbeuteverbesserungen erzielt werden, wobei der Einsatz von natürlichen Trägern mit großer, innerer Oberfläche bzw. der Einsatz von Kunststoffträgern in Abhängigkeit von den herzustellenden Stoffwechselprodukten in gleicher Weise verbesserte Ausbeuten ergibt.

Für eine möglichst rasche Verfahrens Führung bei gleichzeitig hoher Ausbeute ist die Erfindung dahingehend weitergebildet, daß die Herstellung bei Temperaturen zwischen 3 und 45 °C, insbesondere zwischen 10 un 35 °C, durchgeführt wird. In diesem Zusammenhang hat es sich teilweise als günstig erwiesen, daß ein Herstellungsverfahren nicht immer bei gleichbleibender Temperatur geführt wird, sondern daß auch Temperaturwechsel innerhalb der angegebenen Grenzen zu Ausbeuteverbesserungen bzw. Beschleunigung der Reaktionen bzw. Umsätze führen können.

Um ein möglichst reines Endprodukt zu erzielen, wird das erfindungsgemäße Verfahren so geführt, daß die isotopenmarkierten, sekundären Stoffwechselprodukte aus dem flüssigen, synthetischen Kulturmedium durch Extraktion und Aufkonzentration, beispielsweise durch die Kombination der Schritte, wie Fest/Flüssig-Flüssig/Flüssig-Extraktion, zentrifugieren, Filtration, Eindampfen, Einengen und Verdampfen, gewonnen werden. Nach der Gewinnung der isotopenmarkierten, sekundären Stoffwechselprodukte hat es sich als vorteilhaft erwiesen, diese Produkte einer weiteren Reinigung zu unterwerfen, wobei gemäß der Erfindung in bevorzugter weise als Reinigungsverfahren chromatographische Verfahren, insbesondere Säulenchromatografie, präparative Dünnschichtchromatografie, Ionenchromatografie, Affinitätschromatografie, Ausschlußchromatografie und/oder präparative Hochleistungs-Flüssigkeitschromatografie, eingesetzt werden. Nach einem derartigen Aufarbeitungsverfahren und Reinigungsverfahren gelingt es, isotopenmarkierte, sekundäre Stoffwechselprodukte von Pilzen und Bakterien zu erzielen, in welchen wenigstens 95 % der Kohlenstoffatome, Stickstoffatome und/oder Schwefelatome durch die entsprechenden stabilen Isotope ersetzt sind, und somit können Produkte erzielt werden, die eine entsprechende Massendifferenz zum natürlichen Analyten aufweisen, um beispiel weise in einer- Flüssigkeitschromatografie mit massenspektrometrischer Detektion (LCMS) ausreichend von natürlich auftretenden, schweren Isotopen unterscheidbar zu sein und somit beispielsweise einen stabilen, eindeutig identifizierbaren, internen Standard in derartigen Analysen zur Verfügung stellen können.

Die Erfindung zielt weiters auf ein isotopenmarkiertes, sekundäres Stoffwechselprodukt gewählt aus der Gruppe Mycotoxine oder Antibiotika ab, welches im wesentlichen vollständig, insbesondere wenigstens 95 % aller Kohlenstoffatome, Stickstoffatome und/oder Schwefelatome durch stabile Isotope ersetzt aufweist.

Derartige isotopenmarkierte, sekundäre Stoffwechselprodukte, in welchen wenigstens 95 % der Kohlenstoffatome, Stickstoffatome und/oder Schwefelatome durch die entsprechenden stabilen Isotope ersetzt sind, gewählt aus der Gruppe Mycotoxine oder Antibiotika können gemäß einer Weiterbildung der Erfindung als interne Standards in der Analytik, für Stoffwechselstudien in Fütterungsversuchen bei Tieren, für metabolische Studien, zur Aufklärung von Stoffwechselkreisläufen, Abbauwegen und/oder Abbauzeitspannen sowie Einlagerungen eingesetzt werden. In sämtlichen genannten Einsatzzwecken ist es von wesentlicher Bedeutung, einen stabilen und eindeutig nachweisbaren Standard bzw. eine eindeutig nachweisbare und verfolgbare Substanz in dem Versuchsschema bzw. Abbauschema enthalten zu haben, um die einzelnen Verfahrens- bzw. Ablaufschritte eindeutig nachvollziehen zu können.

Gemäß einer Weiterbildung sind in den analytischen Verfahren bzw. den Stoffwechselstudien, Abbauwegen und dgl. als Mykotoxine Trichothecene, wie Nivalenol, Deoxynivalenol, 3-Acetyl-Deoxynivalenol, 15-acetyl, Fusarenon X, T-2 Toxin, HT-2 Toxin, DAS, Fumonisine, wie Fumonisin B1, B2 oder B3, Ochratoxine, wie Ochratoxin A, B, C oder D, Zearalenone, Moniliformin oder Aflatoxine, wie Aflatoxin B1, B2 G1 oder G2, eingesetzt. Mykotoxine sind von immer größerer Bedeutung in der Ätiologie von tierischen Krankheilten und es ist notwendig, ausreichende Mengen derartiger Substanzen technisch herzustellen, um mit chemisch möglichst eindeutigen bzw. reinen Stoffen in der Folge entsprechende toxikologische, veterinärmedizinische Untersuchungen durchführen zu können. Da Mykotoxine eine ernsthafte Gesundheitsgefährdung von Tier un Mensch darstellen, ist deren Analytik ein Thema von globalem Interesse, da insbesondere bereits viele Länder Rich- und Grenzwerte für die Toleranz derartiger Substanzen entwickelt haben. Der Nachweis bzw. die Quantifizierung derartiger Mykotoxine über den Einsatz von internen Standards, welche eindeutig nachweisbar sind und somit eine quantitative Analyse des jeweiligen Toxins ermöglichen, stellt einen bedeutenden Fortschritt beim Nachweis derartiger gefährlicher Substanzen zur Verfügung.

In gleicher Weise hat der Einsatz von Antibiotika, wie die von Actinomyceten gebildeten Antibiotika, wie Tetracycline, Streptomycine oder Aminoglycoside, vo Bazillus sp. gebildete Antibiotika, wie Bacitracin oder Polymyxin, von Penizillium gebildete Antibiotika, wie Penicillin oder Griseofulvin, oder von Cephalosporium gebildete Cephalosporine, immer größere Bedeutung, insbesondere im Fall von Erkrankungen bzw. im Fall eines Nachweises derartiger Substanzen in Nahrungs- und Genußmitteln, wobei auch für diese Substanzen festzuhalten ist, daß Substanzen, mit welchen ein quantitativer Nachweis der Stoffwechselprodukte, wie Antibiotika, gelingt, von vitalem Interesse für die Allgemeinheit sind.

Gemäß einer Weiterbildung gelangen als Stoffwechselprodukte Reinsubstanzen mit einem Durchmarkierungagrad von ¹³C_{,} ¹⁵N bzw. ³³S oder ³⁴S zur Anwendung, wodurch einerseits eine eindeutige Unterscheidung von den nicht markierten Substanzen bzw. Stoffwechselprodukten möglich ist und andererseits auch eine Unterscheidung von natürlich vorkommenden Isotopen mit Sicherheit gewährleistet wird und schließlich in Analysen bzw. Nachweisverfahren eine eindeutig verfolgbare Substanz zur Verfügung gestellt werden kann.

Die Erfindung wird nachfolgend anhand von Beispielen näher erläutert, welche die Herstellung von hochgradig isotopenmarkierten Stoffwechselprodukten zeigen.

### Beispiel 1

### Herstellung von hochgradig isotopenmarkiertem [U-¹³C₁₅], Deoxynivalenol (DON)

Zur Herstellung von vollständig durchmarkiertem ¹³C-DON wird ein Fusarium-Pilz, nämlich Fusarium graminearum, auf inertem Trägermaterial, nämlich Vermiculit, inokuliert und in einem synthetischem Kulturmedium, bestehend aus 0,5 g K₂HPO₄ , 2,0 g NaNO₃, 0,7 g MgSO₄. 7H₂O, 2,0 g KCl, 15 g D-[U-¹³C₆] Glucose, 1,5 g NH₄H₂PO₄, 15 mg Fe (II) SO₄*7H₂O oder 20 mg ZnSO₄*7H₂O, das D-[U-¹³C₆] Glucose als alleinige Kohlenstoffquelle enthält, bebrütet. Nach 5 Wochen bei ca. 28°C wird das toxinhältige Material mit Ethylacetat extrahiert und anschließend zur Standardqualität (Reinheit > 98 %) mittels Extraktion, Chromatografie und Kristallisation aufgereinigt.

Pro Produktionsansatz werden ca. 40 ml Inkubationsansatz bereitet. Gepooltes, toxinhältiges Material wird anschließend weiterverarbeitet. Aus 1000 ml Ansatz werden zwischen 5 und 50 mg [7.5 bis 17.5mg] vollständig durchmarkiertes [U-¹³C₁₅] -DON gewonnen.

Das gereinigte Produkt wurde mittels folgender Analyseverfahren charakterisiert: ¹H NMR u. ¹³C-NMR

LC-MS/MS Q Trap zur Bestimmung des ¹³C Isotopenanteils Reinheits- und Konzentrationsbestimmung gegen Referenzmaterialien mit UV/VIS und HPLC-DAD Justierung der Konzentration

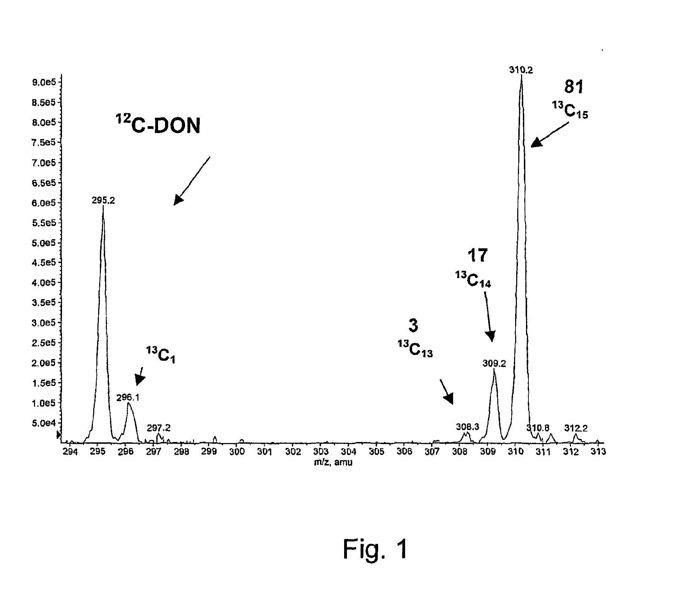

Qualitätskontrolle mit UV/VIS, HPLC-DAD, LC-MS/MS Q Trap Ein derartiges hochgradig isotopenmarkiertes ¹³C₁₅-Deoxynivalenol (¹³C₁₅-DON) kann beispielsweise als interner Standard eingesetzt werden. Ein derartiger interner Standard hat eine exakt um 15 g/Mol schwerere Molmasse und somit erscheint sein Signal im Massenspektrum (Fig. 1) genau 15 amu höher als das Signal des Analyten. Da sämtliche sonstigen chemischen und physikalischen Eigenschaften des ¹³C-markierten DON ident mit dem Analyten sind, geht ein derartiger interner Standard exakt dieselbe Fragmentierung wie der Analyt ein, auch die Ionisierung der Substanz ist gleich und somit auch die Ionisierungsausbeute. Dies bedeutet, daß die Signalhöhe der Fragmente bzw. Substanzionen zwischen internem Standard und Analyten absolut vergleichbar wird und, da die Konzentration des internen Standards in einer Analyse bekannt ist, können dadurch direkte Rückschlüsse auf die Konzentration eines Analyten gezogen werden, wodurch ein derartig hochgradig durchmarkiertes Deoxynivalenol einen nahezu idealen internen Standard darstellt.

Fig. 1 zeigt C₁₂-DON und C₁₃-DON in einem gemeinsamen Massenspektrum. In Fig. 1 ist neben den Molekülpeaks von ¹³C₁₅-DON und jenem von ¹²C-DON bei 295,2 bzw. 310,2 auch die Verteilung der Verbindungen, bei denen nicht alle C-Atome durchmarkiert wurden und somit nicht aus einer Isotopensorte bestehen (Isotopomere), gezeigt. Im Fall des natürlich vorkommenden Deoxynivalenols ist dies das ¹³C₁-DON, was der natürlichen Verteilung zwischen C₁₂ und C₁₃ entspricht.

### Beispiel 2

### Herstellung von hochgradig isotopenmarkiertem ¹³C-Fumonisin

Zur Herstellung eines vollständig mit ¹³C markierten Fumonisins werden 1000 ml Flüssigmedium, bestehend aus 0,5 g KH₂PO₄, 0,5 g KNO₃, 0,7 g MgSO₄.7H₂O, 2, 0 g KCl, 17, 5 g D-[U-¹³C₆] Glucose, 1,5 g NH₄H₂PO₄, 15 mg Fe (II) SO₄*7H₂O und 20 mg ZnSO₄*7H₂O, mit D-[U-¹³C₆] Glucose als einziger Kohlenstoffquelle, aufgebracht auf 1 x 1 x 1 cm große Schaumstoffwürfel, mit Fusarium moniliforme inokuliert und bei 28 °C und 70 % relativer Luftfeuchtigkeit im Brutschrank bebrütet. Nach 3 Wochen wird das toxinhältige Material mit einem Lösungsmittelgemisch aus 1:1 Acetonitril: H₂O extrahiert und anschließend zur Standardqualität (Reinheit > 98 %) mittels Extraktion und Chromatografieschritte, wie Ionentauscherchromatografie, Flash-Säulenchromatografie mit Kieselgel, Dünnschichtchromatografie und präparative HPLC, aufgereinigt.

Pro 1000 ml Ansatz werden so 80 - 240 mg an ¹³C-Fumonisinen gewonnen (HPLC-FLD).

### Beispiel 3

### Herstellung von hochgradig isotopenmarkiertem [U-¹³C₁₇] -3-Acetyl-Deoxynivalenol

Zur Herstellung von hochgradig isotopenmarkiertem [U₋¹³C₁₇]-3-Acetyl-Deoxynivalenol werden 1000 ml synthetisches Flüssigmedium, bestehend aus 0,5 g KH₂PO₄, 0,5 g KNO₃, 0,7 g MgSO₄*7H₂O, 2,0 g KCl, 17,5 g D- [U-¹³C₆] Glucose, 1,5 g NH₄H₂PO₄, 15 mg Fe(II)SO₄*7H₂O und 20 mg ZnSO₄*7H₂O, das vollständig isotopenmarkierte [U-¹³C₆]-Glucose als alleinige Kohlenstoffquelle enthält, aufgebracht auf eine Diatomeenerde, nämlich Isolute HM-N, mit Fusarium graminearum inokuliert und bei 28 °C für 9 Tage im Brutschrank bebrütet. Nach 9 Tagen wird das toxinhältige Material geerntet, mit Acetonitril/H₂O-Aceotrop extrahiert und anschließend zur Standardqualität (Reinheit > 98 %) mittels Extraktion, Chromatografie, Kristallisation, Büchi-MPLC und Umkristallisieren aufgereinigt. Aus einem Ansatz können ca. 15 - 50 mg hochreines Endprodukt gewonnen werden. Die Kontrolle der Reinheit erfolgte durch LC-UV Analyse mit einer C18-Kapillarsäule.

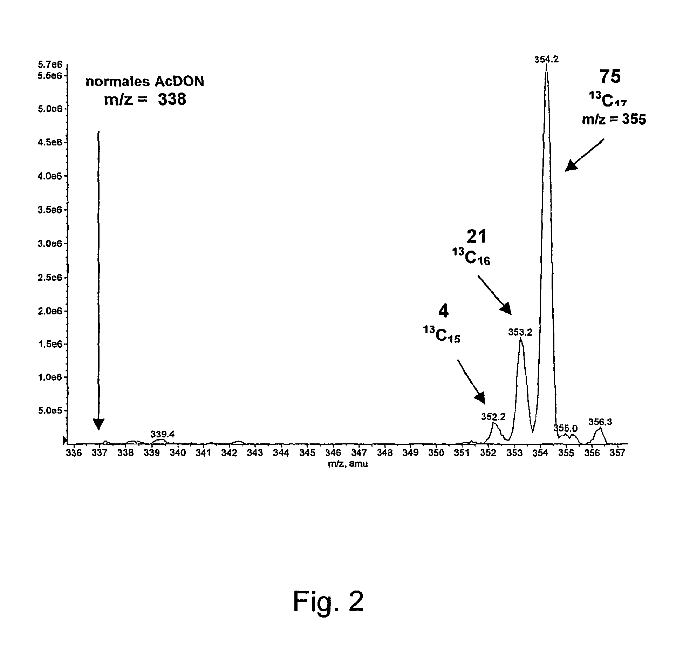

Ein derartiges hergestelltes, isotopenmarkiertes 3-Acetyl-Deoxynivalenol hat eine gegenüber dem nicht durchmarkierten 3-Acetyl-Deoxynivalenol um 17 g/Mol schwerere Molekülmasse. Nicht durchmarkiertes 3-Acetyl-Deoxynivalenol hat eine Molekülmasse von M/z = 338 und das vollständig isotopenmarkierte Produkt hat eine Molekülmasse von 355. Fig. 2 zeigt das Massenspektrum des reinen ¹³C-3-Acetyl-Deoxynivalenol, bei welchem zu erkennen ist, daß eine Durchmarkierung des Produkts zu 75 % gelungen ist und die Isotopenverteilung des Produkts erkennbar ist. Die Verteilung des Produkts und der nicht vollständig durchmarkierten Isotopomere ist in diesem Fall von der Isotopenreinheit des Ausgangsprodukts ¹³C₆-Glukose abhängig und kann bei Einsatz von vollständig reiner ¹³C₆-Glukose noch deutlich in Richtung vollständig durchmarkiertes Produkt verschoben werden. Aus Fig. 2 ist jedoch deutlich zu erkennen, daß Isotopomere, die weniger als 13 ¹³C-Atome besitzen, bereits so gut wie nicht vorhanden sind, so daß auch das ¹³C-3-Acetyl-Deoxynivalenol sehr gut als interner Standard eingesetzt werden kann.

### Beispiel 4

### Herstellung von hochgradig isotopenmarkiertem [U-¹³C₁₇] -15-Acetyl-Deoxynivalenol

Zur Herstellung von hochgradig isotopenmarkiertem [U-¹³C₁₇]-15-Acetyl-Deoxynivalenol wird Kulturmedium, bestehend aus 0,5 g K₂HPO₄, 2,0 g NaNO₃, 0,7 g MgSO₄ * 7H₂O, 2,0 g KCl, 15 g D-[U-¹³C₆] Glucose, 1,5 g NH₄H₂PO₄, 15 mg Fe(II)SO₄*7H₂O und 20 mg ZnSO₄*7H₂O, auf grobkörnigem Phyllosilikatträger mit Fusarium graminearum inokuliert und im Brutschrank bei 28 °C bebrütet. Nach 9 Tagen wird das toxinhältige Material geerntet, mit Ethylacetat extrahiert und anschließend zur Standardqualität (Reinheit > 98 %) mittels Extraktion, Chromatografie und Kristallisation aufgereinigt. Alternativ zur Kristallisation kann auch ein weiterer Reinigungsschritt mittels präparativer HPLC eingesetzt werden.

Aus einem Fermentationsansatz können ca. 30 - 60 mg hochreines Zielprodukt gewonnen werden.

### Beispiel 5

### Herstellung von hochgradig isotopenmarkiertem [U-¹³C₁₅]-Nivalenol oder [U-¹³C₁₇] Fusarenon X

Zur Herstellung von hochgradig isotopenmarkiertem [U-¹³C₁₅]-Nivalenol oder [U-¹³C₁₇] Fusarenon X wird ein Flüssigmedium, bestehend aus 0,5 g K₂HPO₄, 2,0 g NaNO₃, 0,7 g MgSO₄*7H₂O, 2,0 g KCl , 15 g D- [U-¹³C₆] Glucose , 1,5 g NH₄H₂PO₄, 15 mg Fe(II)SO₄*7H₂O oder 20 mg ZnSO₄*7H₂O, mit [U-¹³C₆]-Glucose als einziger Kohlenstoffquelle und inertes Phyllosilikat mit Fusarium nivale inokuliert und bei 28 °C für 5 Wochen bebrütet. Dann wird das toxinhältige Material mit Methanol und Methylenchlorid extrahiert und anschließend zur Standardqualität (Reinheit > 98 %) mittels Extraktion, Chromatografie und Kristallisation aufgereinigt. Alternativ zur Kristallisation kann auch ein weiterer Reinigungsschritt mittels präparativer HPLC eingesetzt werden.

### Beispiel 6

### Herstellung von hochgradig isotopenm-arkiertem [U-¹³C₂₀]-Ochratoxin A

Zur Gewinnung der Zielsubstanz wird der Pilz Petromyces albertensis auf einem inertem Phyllosilikatträger mit synthetischem Flüssigmedium, bestehend aus 0,5 g K₂HPO₄, 2,0 g NaNO₃, 0,7 g MgSO₄*7H₂O, 2,0 g KCl, 15 g D- [U-¹³C₆] Glucose, 1, 5 g NH₄H₂PO₄, 15 mg Fe(II)SO₄*7H₂O oder 20 mg ZnSO₄* 7H₂O, das als einzige Kohlenstoffquelle vollständig ¹³C-markierte Glucose enthält, fermentiert. Die Kolben werden in der Folge in einem Brutschrank 6 Wochen bei 28 °C und 70 % Luftfeuchte inkubiert und danach mit Toluol extrahiert. Die Zielsubstanz wird wie in den vorherigen Beispielen säulenchromatographisch aufgereinigt und umkristallisiert.

### Beispiel 7

### Herstellung von hochgradig isotopenmarkiertem [U-¹³C₁₈]-, Zearalenon

Zur Herstellung von hochgradig isotopenmarkiertem [U-¹³C₁₈]-Zearalenon werden 1000 ml Flüssigmedium, bestehend aus 0,5 g KH₂PO₄, 0,5 g KNO₃, 0,7 g MgSO₄*7H₂O, 2,0 g KCl, 17,5 g D-[U-¹³C₆] Glucose, 1, 5 g NH₄H₂PO4, 15 mg Fe(II)SO₄*7H₂O und 20 mg ZnSO₄*7H₂O, mit D- [U-¹³C₆] Glucose als einziger Kohlenstoffquelle, aufgebracht auf porösem, gebranntem Ton in Granulatform, nämlich Seramis oder Lecca, mit Fusarium semitectum inokuliert und bei 28 °C und 70 % relativer Luftfeuchtigkeit im Brutschrank bebrütet. Nach 3 Wochen wird das toxinhältige Material mit reinem Petrolether und Petrolether/Ethylacetat-Gemisch von 4:1 und 2:1 extrahiert und anschließend zur Standardqualität (Reinheit > 98 %) mittels Extraktion und Chromatografieschritten, wie Ionentauscherchromatografie, Flash-Säulenchromatografie mit Kieselgel, Dünnschichtchromatografie und präparativer HPLC, aufgereinigt.

### Beispiel 8

### Herstellung von hochgradig isotopenmarkiertem ¹⁵N₅-Rocquefortine C

Zur Herstellung eines vollständig mit dem Stickstoffisotop ¹⁵N markierten Rocquefortin C werden 1000 mL Flüssigmedium, bestehend aus 0,8 g KH₂PO₄, 0,7 g MgSO₄*7H₂O, 1,0 g KCl, 17,5 g D-Glucose, 1,0 g ¹⁵NH₄¹⁵NO₃, 1,5 g NaH₂PO₄, 15 mg Fe (II) SO₄*7H₂O, 20 mg ZnSO₄*7H₂O, mit ¹⁵NH₄¹⁵NO₃ als einziger Stickstoffquelle, aufgebracht auf groben Kieselgur, mit Penicillium commune inokuliert und bei 12 °C und 70 % Luftfeuchtigkeit im Brutschrank bebrütet. Nach 48 Tagen wird das toxinhaltige Material mit einem organischen Lösungsmittelgemisch, bestehend aus 9:1 Chloroform:Methanol extrahiert und anschließend zur Standardqualität (Reinheit > 98 %) mittels Flüssig-Flüssig-Extraktion, Flash-Säulenchromatographie mit Kieselgel und präparativer HPLC, aufgereinigt. Pro 1000 mL Ansatz werden 300 mg an ¹⁵N₅-Rocquefortine C gewonnen (HPLC-FLD).

### Beispiel 9

### Herstellung von hochgradig isotopenmarkiertem ¹⁵N₂-³³S-Penicillin

Zur Herstellung eines vollständig mit dem Stickstoffisotop ¹⁵N und dem Schwefelisotop ³³S markierten Penicillin werden 1000 mL Flüssigmedium, bestehend aus 1,0 g K₂HPO₄, 0,2 g MgCl₂, 20,0 g D-Glucose, 1,0 g ¹⁵NH₄¹⁵NO₃, 0,5 g Na₂³³SO₄, 1,5 g Na₂HPO₄, 5 mg Fe(II)Cl₂, 5 mg ZnCl₂, mit ¹⁵NH₄¹⁵NO₃ als einziger Stickstoffquelle und Na₂³³SO₄ als einzige Schwefelquelle, aufgebracht auf kleinen Schaumstoffwürfeln, mit Penicillium notatum inokuliert und bei 28 °C und 70 % Luftfeuchtigkeit im Brutschrank bebrütet. Nach 30 Tagen wird das toxinhaltige Material mit Ethylacetat extrahiert und anschließend zur Standardqualität (Reinheit > 98 %) mittels Flüssig-Flüssig-Extraktion, Flash-Säulenchromatographie mit Kieselgel und präparativer HPLC, aufgereinigt. Pro 1000 mL Ansatz werden 500 mg an ¹⁵N₂-³³S-Penicillin gewonnen (HPLC-FLD).

## Patentansprüche

1. Verfahren zur Herstellung von isotopenmarkierten, sekundären Stoffwechselprodukten von Pilzen oder Bakterien zur Herstellung eines internen Standards in der Analytik, für Stoffwechselstudien in Fütterungsversuchen bei Tieren, für metabolische Studien, zur Aufklärung von Stoffwechselkreisläufen, Abbauwegen und/oder Abbauzeitspannen sowie Einlagerungen in einem flüssigen, synthetischen Kulturmedium, **dadurch gekennzeichnet, daß** die Synthese durch Immobilisieren der Pilze oder Bakterien auf einem inerten Träger unter Zugabe von flüssigem, synthetischem Kulturmedium, in welchem wenigstens 95 % der Kohlenstoffatome, Stickstoffatome und/ oder Schwefelatome durch stabile Isotope ersetzt wurden, durchgeführt wird.

2. Verfahren nach Anspruch 1, **dadurch gekennzeichnet, daß** als Kohlenstoffquelle im flüssigen, synthetischen Kulturmedium Zucker oder Zuckeralkohole, insbesondere D-[U-¹³C₆]-Glucose, ¹³C-Saccharose, ¹³C-Glycerin und/oder ¹³C-Acotat, als Stickstoffquellen ¹⁵N-Aminosäuren, -Nitrate, -Ammoniumverbindungen oder -Harnstoff, als Schwefelquellen ³³S- oder ³⁴S-Sulfate, -Sulfide oder -Aminosäuren eingesetzt sind.

3. Verfahren nach Anspruch 1 oder 2, **dadurch gekennzeichnet, daß** das flüssige, synthetische Kulturmedium zusätzlich eine Mischung, gewählt aus anorganischen Salzen oder Säuren und Basen mit den Ionen N^{a+}, K⁻, ca⁺⁺, Mg⁺⁺, Fe⁺⁺⁺, Zn⁺⁺, Cu⁺⁺, B⁺⁺⁺ sowie CO₃⁻⁻, SO₄⁻⁻, PO₄⁻⁻⁻, NO₃⁻, enthält.

4. verfahren nach Anspruch 1, 2 oder 3, **dadurch gekennzeichnet, daß** als inerter Träger ein natürlicher oder synthetischer Träger mit großer innerer Oberfläche, insbesondere Silikat, Schichtsilikat, Zeolith, Bentonit, gebrannter Ton, Diatomeenerde, Kunststoffe oder dgl., eingesetzt werden.

5. verfahren nach Anspruch 4, **dadurch gekennzeichnet, daß** als inerter Träger ein Aluminiumsilikat, beispielsweise ein Zeolith oder ein Schichtsilikat, insbesondere ein vermiculit, aus der Gruppe der Glimmerminerale, in natürlicher oder behandelter Form eingesetzt wird.

6. Verfahren nach Anspruch 4, **dadurch gekennzeichnet, daß** als inerter Kunststoffträger Schaumstoff, Polyamid, Silikon, Polyethylen, Polypropylen, Polytetrafluorethylen, Polyester oder dgl. eingesetzt wird.

7. Verfahren nach einem der Ansprüche 1 bis 6, **dadurch gekennzeichnet, daß** die Herstellung bei Temperaturen zwischen 3 und 45 °C, insbesondere zwischen 10 und 35 °C, durchgeführt wird.

8. Verfahren nach einem der Ansprüche 1 bis 7, **dadurch gekennzeichnet, daß** die isotopenmarkierten, sekundären Stoffwechselprodukte aus dem flüssigen, synthetischen Kulturmedium durch Extraktion und Aufkonzentration, beispielsweise durch die Kombination der Schritte, wie Fest/Flüssig-Flüssig/Flüssig-Extraktion, Zentrifugieren, Filtration, Eindampfen, Einengen und Verdampfen, gewonnen werden.

9. Verfahren nach Anspruch 8, **dadurch gekennzeichnet, daß** als Reinigungsverfahren chromatographische Verfahren, insbesondere Säulenchromatografie, präparative Dünnschichtchromatografie, Iononchromatografie Affinitätschromatografie, Ausschlußchromatografie und/oder präparative Hochleistungs-Flüssigkeitschromatografie, eingesetzt werden.

10. Isotopenmarkiertes, sekundäres Stoffwechselprodukt, in welchem wenigstens 95 % der Kohlenstoffatome, Stickstoffatome und/oder Schwefelatome durch die entsprechenden stabilen Isotope ersetzt sind, gewählt aus der Gruppe Mycotoxine, Trichothecene, wie Nivalenol, Deoxynivalenol, 3-Acetyl-Deoxynivalenol, 15-Acetyl-Deoxynivalenol, Fusarenon X, T-2 Toxin, HT-2 Toxin, DAS, Fumonisine, wie Fumonisin B1, B2 oder B3, Ochratoxine, wie Ochratoxin A, B, C oder D, Zearalenone, Moniliformin oder Aflatoxine, wie Aflatoxin B1, B2 G1 oder G2 oder Antibiotika, die von Actinomyceten gebildeten Antibiotika, wie Tetracycline, Streptomycine oder Aminoglycoside, von Bazillus sp. gebildete Antibiotika, wie Bacitracin oder Polymyxin, von Penicillium gebildete Antibiotika, wie Penicillin oder Griseofulvin, oder von Cephalosporium gebildete Cephalosporine, welches gemäß einem Verfahren nach einem der Ansprüche 1 bis 9 hergestellt ist, zur Herstellung eines internen Standards in der Analytik, für Stoffwechselstudien in Fütterungsversuchen bei Tieren, für metabolische Studien, zur Aufklärung von Stoffwechselkreisläufen, Abbauwegen und/oder Abbauzeitspannen sowie Einlagerungen.

11. Stoffwechselprodukt nach Anspruch 10, als Reinsubstanz mit einem Durchmarkierungsgrad von ¹³C, ¹⁵N, ³³S oder ³⁴S von wenigstens 95 %.

## Claims

1. A method for producing isotopically labelled secondary metabolic products of fungi or bacteria for the production of an internal standard in analytics, for metabolic studies in animal feeding tests, for metabolic studies, for clarifying metabolic cycles, degradation paths and/or degradation periods as well as intercalations in a liquid synthetic culture medium, **characterized in that** the synthesis is carried out by immobilizing the fungi or bacteria on an inert carrier while adding a liquid synthetic culture medium in which at least 95% of the carbon atoms, nitrogen atoms and/or sulphur atoms have been replaced by stable isotopes.

2. A method according to claim 1, **characterized in that** sugars or sugar alcohols, in particular D- [U-¹³C₆] - glucose, ¹³C -sucrose, ¹³C-gycerol and/or ¹³C-acetate are used as carbon sources in the liquid synthetic culture medium, ¹⁵N-amino acids, -nitrates, -ammonium compounds or -urea are used as nitrogen sources, ³³S_ or ³⁴S-sulphates, -sulphides or -amino acids are used as sulphur sources.

3. A method according to claim 1 or 2, **characterized in that** the liquid synthetic culture medium additionally contains a mixture selected from inorganic salts or acids and bases having the ions Na⁺, K⁺, Ca⁺⁺, Mg⁺⁺, Fe⁺⁺⁺ Zn⁺⁺, Cu⁺⁺, B⁺⁺⁺ as well as CO₃⁻⁻, SO₄⁻⁻, PO₄⁻⁻⁻, NO₃⁻.

4. A method according to claim 1, 2 or 3, **characterized in that** a natural or synthetic carrier having a large internal surface area, in particular silicate, layered silicate, zeolite, bentonite, burnt clay, diatomaceous earth, synthetics or the like, is used as said inert carrier.

5. A method according to claim 4, **characterized in that** an aluminium silicate, for instance a zeolite or a layered silicate, in particular a vermiculite, from the group of mica minerals is used in natural or treated form as said inert carrier.

6. A method according to claim 4, **characterized in that** foamed materials, polyamide, silicone, polyethylene, polypropylene, polytetrafluoroethylene, polyester or the like are used as said inert synthetic carrier.

7. A method according to any one of claims 1 to 6 **characterized in that** the production is realized at temperatures ranging between 3 and 45 °C, in particular between 10 and 35 °C.

8. A method according to any one of claims 1 to 7 **characterized in that** the isotopically laballed secondary metabolic products are recovered from the liquid synthetic culture medium by extraction and concentration, for instance by a combination of steps like solid/liquid -liquid/liquid extraction, centrifugation, filtration and evaporation.

9. A method according to claim 8, **characterized in that** chromatographic methods and, in particular, column chromatography, preparative thin-layer chromatography, ion chromatography, affinity chromatography, exclusion chromatography and/or preparative high-pressure liquid chromatography are used as purification processes.

10. An isotopically labelled secondary metabolic product, in which at least 95% of the carbon atoms, nitrogen atoms and/or sulphur atoms have been replaced by stable isotopes, selected from the group of mycotoxins, trichothecenes such as nivalenol, deoxynivalenol, 3-acetyl-deoxynivalenol, 15-acetyl deoxynivalenol, fusarenon X, T-2 toxin, HT-2 toxin, DAS, fumonisins, such as fumonisin, B1, B2 or B3, ochratoxins, such as ochratoxin A, B, C or D, zearalenones, moniliformin or aflatoxins such as aflatoxin B1, B2, G1 or G2 or antibiotics such as antibiotics formed of actinomycetes, like tetracyclines, streptomycines or aminoglycosides, antibiotics formed of Bazillus sp., like bacitracin or polymyxin, antibiotics formed of penicillium, like penicillin or griseofulvin, or cephalosporins formed of cephalosporium which is produced according to a method of any of claims 1 to 9 for the production of an internal standard in analytics, for metabolic studies in animal feeding tests, for metabolic studies, for clarifying metabolic cycles, degradation paths and/or degradation periods as well as intercalations.

11. A metabolic product according to claim 10 as a pure substance having a labelling degree with ¹³C, ¹⁵N-, ¹³S- or ³⁴S of at least 95%.

## Revendications

1. Procédé de production de métabolites secondaires marqués par isotope, issus de champignons ou de bactéries, pour la production d'une norme interne en chimie analytique, pour des études métaboliques dans des essais alimentaires chez les animaux, pour des études métaboliques, pour expliquer les circuits métaboliques, les voies de décomposition et/ou les durées de décomposition et les stockages dans un milieu de culture liquide synthétique, **caractérisé en ce que** la synthèse est réalisée par immobilisation des champignons ou bactéries sur un support inerte en ajoutant un milieu de culture fluide synthétique, dans lequel au moins 95 % des atomes de carbone, des atomes d'azote et/ou des atomes de soufre ont été remplacés par des isotopes stables.

2. Procédé selon la revendication 1, **caractérisé en ce que** l'on utilise comme source de carbone dans le milieu de culture fluide synthétique, du sucre ou des alcools de sucre, en particulier du glucose D- (U-¹³C₆), du saccharose ¹³C, de la glycérine ¹³C et/ou de l'acétate ¹³C, comme sources d'azote, des acides aminés ¹⁵N, des nitrates ¹⁵N, des composés d'ammonium ¹⁵N ou de l'urée ¹⁵N, comme sources de soufre, des sulfates ³³S ou ³⁴S, des sulfures ³⁴S ou des acides aminés ³⁴S.

3. Procédé selon la revendication 1 ou 2, **caractérisé en ce que** le milieu de culture fluide synthétique comprend en outre un mélange choisi parmi les sels anorganiques ou les acides et bases avec des ions Na⁺, K⁺, Ca⁺⁺, Mg⁺⁺, Fe⁺⁺⁺, Zn⁺⁺ Cu⁺⁺, B⁺⁺⁺ et CO₃⁻⁻, SO₄⁻⁻, PO₄⁻⁻, NO₃⁻.

4. Procédé selon la revendication 1, 2 ou 3, **caractérisé en ce que** l'on utilise comme support inerte, un support naturel ou synthétique présentant une grande surface interne, en particulier, du silicate, du phyllosilicate, de la zéolithe, de la bentonite, de la terre cuite, de la terre de diatomée, des matières plastiques ou similaires.

5. Procédé selon la revendication 4, **caractérisé en ce que** l'on utilise comme support inerte, un silicate d'aluminium, par exemple, une zéolithe ou un phyllosilicate, en particulier une vermiculite, du groupe des minéraux mica, sous forme naturelle ou traitée.

6. Procédé selon la revendication 4, **caractérisé en ce que** l'on utilise comme support synthétique inerte, de la mousse, du polyamide, de la silicone, du polyéthylène, du polypropylène, du polytétra-fluoroéthylène, du polyester ou similaire.

7. Procédé selon l'une des revendications 1 à 6, **caractérisé en ce que** la production est réalisée à des températures entre 3 et 45° C, en particulier entre 10 et 35° C.

8. Procédé selon l'une des revendications 1 à 7, **caractérisé en ce que** les métabolites secondaires marqués par isotope du milieu de culture synthétique fluide sont obtenus par extraction et concentration, par exemple, par la combinaison des étapes telles que l'extraction solide/liquide-liquide/liquide, la centrifugation, la filtration, l'évaporation, la concentration et vaporisation.

9. Procédé selon la revendication 8, **caractérisé en ce que** l'on utilise comme procédé de purification, un procédé chromatographique, en particulier la chromatographie en colonne, la chromatographie préparatoire en couche mince, la chromatographie ionique, la chromatographie d'affinité, la chromatographie d'exclusion et/ou la chromatographie préparatoire en phase liquide haute performance.

10. Métabolite secondaire marqué par isotope, dans lequel au moins 95% des atomes de carbone, des atomes d'azote et/ou des atomes de soufre sont remplacés par les isotopes stables correspondants, choisi dans le groupe des mycotoxines, des trichothécènes tels que nivalénol, désoxynivalénol, 3-acétyl-désoxynivalénol, 15-acétyl-désoxynivalénol, fusarénone X, toxine T-2, toxine HT-2, DAS, des fumonisines telle que la fumonisine B1, B2 ou B3, des ochratoxines telles que l'ochratoxine A, B, C ou D, des zéaralénones, de la moniliformine ou des aflatoxines telles que l'aflatoxine B1, B2, G1 ou G2 ou des antibiotiques tels que les antibiotiques formés par des actinomycètes, tels que les tétracyclines, les streptomycines ou les aminoglycosides, des antibiotiques formés à partir de *Bacillus sp* tels que la bacitracine ou la polymyxine, des antibiotiques formés à partir de pénicilline tels que la pénicilline ou la griséofulvine ou des céphalosporines formées à partir de Cephalosporium qui est produit selon un procédé selon l'une des revendications 1 à 9, pour la production d'une norme interne en chimie analytique, pour des études métaboliques dans des essais alimentaires chez les animaux, pour des études métaboliques, pour explorer les circuits métaboliques, les voies de décomposition et/ou les durées de décomposition et les stockages.

11. Métabolite selon la revendication 10, comme substance pure avec un degré de marquage de ¹³C, ¹⁵N, ³³S ou ³⁴S d'au moins 95%.
